# EUROPEAN PATENT APPLICATION

(11) **EP 2 371 282 A1**
(43) Date of publication of application: **05.10.2011**
(21) Application number: 10809582.9
(22) Date of filing: 05.05.2010
(51) Int. Cl.: A61B 5/11

(54) **DEVICE FOR MEASURING THE ROTARY INSTABILITY OF THE KNEE**

(30) Priority: 18.08.2009 ES 200930607
(71) Applicant: Fundacion Garcia Cugat, 08006 Barcelona (ES)
(72) Inventor: CUGAT BERTOMEU, Ramón, E-08213 Polinya (Barcelona) (ES)
(74) Representative: Durán Moya, Luis-Alfonso
(86) International application number: PCT/ES2010/000195
(87) International publication number: WO 2011/020932

(57) **Abstract**

The invention relates to a device for measuring the rotary instability of the knee. The invention relates to a device for determining the condition of the knee ligaments, **characterised in that** said device includes at least one means for exerting a force that rotates the tibia about the axis thereof, at least one means for measuring the angle of rotation of the tibia, and at least one means for measuring the torque exerted in order to produce said rotation.

## Description

The present invention relates to a device for measuring the instability of the knee for preoperative, interoperative and postoperative purposes.

The present invention, among other things, allows patients with injuries to the posterior cruciate ligament (PCL) to be diagnosed.

One of the present techniques for the diagnosis of patients with a PCL injury is based on the emission of X-rays to obtain a radiograph. This technique does not show the patient's injury exactly but gives an idea of the type of injury. However, it has low accuracy due to its two-dimensionality and the presence of bones which make it difficult to see the tissues. Its interpretation is therefore very complex, making diagnosis impossible or giving rise to mistaken diagnoses.

Another known technique is magnetic resonance imaging (MRI). This technique is a diagnostic process which uses a combination of large magnets, radiofrequencies and a computer to produce detailed images of the organs and structures inside the body; it is usually very efficient for detecting tissue damage. A drawback of this technique is the high cost of using it.

Another known technique is computerised tomography which is based on image diagnosis which uses a combination of X-rays and computer technology to produce both horizontal and vertical transverse images (often known as "slices") of the body. A CT scan shows detailed images of any part of the body, including the bones, muscles, fat and organs. The CT scan is more detailed than normal X-rays and therefore more effective for tissue injuries but it requires a complex interpretation.

Another known technique is diagnostic arthroscopy. This is a method diagnosis which requires invasion of the joint. In this procedure a small illuminated optical tube (arthroscope) is used which is inserted into the joint through a small incision. The images of the inside of the joint are projected onto a screen and used to assess any degenerative and/or arthritic change in the joint.

Another known technique for this type of diagnosis is radionuclide scanning, which consists of a nuclear diagnostic technique by means of images which uses a very small amount of radioactive material which is injected into the patient's bloodstream for detection by a scanner. This examination shows the blood flow to the bone and cellular activity inside said bone so that injuries to the ligament can be detected indirectly.

Another technique consists of manually causing an opposite movement to the action of the ligament so that the health professional can detect a lack of opposing action by the ligament. This process has the drawback that the movement must be very precisely controlled as the injury may be aggravated and in addition it gives rise to many false measurements since this method depends solely on the sensitivity of the health professional and moreover the resilience of the ligament varies for each patient.

The present invention discloses a device which, in addition to diagnosis, allows the injury the patient may have to be quantified (preoperative), the size of the injury and the best treatment for the specific case to be determined (interoperative), and thus the effectiveness of the treatment used and the patient's progress to be determined via periodic measurements (post-operative), provided that the lateral collateral ligament (LCL) is sound.

Given that the present invention is more economical and/or precise than those described above, as well as not being invasive or causing any pain or damage to the patient, it is a method suitable for carrying out periodic measurements and monitoring the injury more effectively.

Accordingly, the present invention discloses a device for determining the condition of the knee ligaments, characterised in that it comprises at least a means of applying force to rotate the tibia about its own axis, at least a means of measuring the angle of rotation of the tibia and at least a means of measuring the momentum of the force applied to produce said rotation.

The operating procedure followed with the device disclosed by the present invention comprises measuring the momentum of the force required to produce a rotation of the tibia about its own axis and taking a reference in order to determine the angle of rotation so as to quantify the rotation produced. On applying rotational force to the tibia, assuming that the muscles are relaxed, the tissue that has a tendency to generate an opposing force is the posterior cruciate ligament (PCL). The method is therefore based on measuring the opposing force which may be produced by the PCL, which is novel with regard to what was known previously. Said force can not only be analysed in absolute terms but can also be compared with the force produced by the PCL of the sound knee to determine with sufficient precision not only whether an injury exists but also the severity thereof. For explanatory purposes and a better understanding, the reference angle is measured as the angle of rotation of the feet about the sagittal line of the leg with the ankle joint locked, although it is possible to use other references.

The device proposed by the present invention also comprises the measurement of the degree of rotation. The object is to relate the force opposing the movement to the angle of rotation from which, combined with the possibility of carrying out various measurements both in the outward (eversion) and inward (inversion) turning directions, the hysteresis of the cycle can be measured. According to the investigations of the inventor, damaged ligaments exhibit hysteresis that can be clearly differentiated from that of sound ligaments, in which hysteresis is almost zero. In addition, because this analysis should not cause the patient any kind of pain or discomfort it preferably has an emergency stop button which the patient presses if there is discomfort. Operating said button should leave the device turning freely and so avoid subsequent damage.

Another object of the present invention is to disclose a device for measuring the rotational instability of the knee allowing preoperative, interoperative and postoperative procedures to be carried out according to the method described above.

The device according to the present invention measures the force required to rotate the tibia preferably outwards (in the opposite direction to the other leg) and to measure the angular position of the reference. For greater convenience in interpreting the results, the device may produce a graph of said response in order, for example, to obtain an acceptable comparison of the behaviour of the PCL of the sound knee and the PCL that is to be diagnosed.

The device described by the present invention preferably takes account of additional factors such as myotatic reflex. A myotatic reflex is the one that occurs when a muscle is stretched and responds with a contraction opposed to the stretching, said contraction being the cause of false data in the procedure. It is therefore necessary to ensure that the load on the ligament is constant. Thus, an advantage of the present invention is the possibility of allowing the PCL to deform slowly and gradually. Accordingly, the device may have a movement controller which is configured to produce a slow, controlled movement which ensures that the result both for the force and for the angle of displacement is one in which the ligament has been given time to undergo maximum deformation.

Further, it is advisable that the muscles be at rest, so that the force applied acts principally on the ligament and has direct effects on the affected area. To achieve this object, preferably electromyography (EMG) equipment may be used to check the resting state of the muscles.

EMG makes possible a neurophysiological examination of the biometric muscular activity which consists of applying small, low-voltage electrodes in the form of needles in the muscle area to be examined (for the purposes of the present invention, the quadriceps area). As muscle tissue is electrically neutral when at rest, zero activity should be produced in the oscilloscope of the EMG equipment in order to make appropriate use of the device according to the present invention.

When the angle through which the feet are moved is taken as the reference angle, it should be borne in mind that the device must have a suitable system for fixing the intermediate joints (in this case, the ankles) to avoid producing false angle readings caused by the movement of said intermediate joints.

In a particularly preferred embodiment, the device comprises in particular:
- two motors to rotate the tibias,
- two force meters,
- two angular position meters,
- a device for displaying results,
- EMG equipment,
- a support for positioning the knees at 90°, and
- a support to prevent rotation of the ankles.

Preferably, to achieve the turning direction required by the present invention electric motors may be used, more preferably with speed regulators connected to the axis of rotation of the tibia in order to produce the corresponding torque.

A traction-compression or bending load cell is used preferably as a force meter, the function of which is to measure continuously the force required for the angular movement of the tibia.

Preferably a potentiometer with a nonlinearity of less than 0.5% is used as an angular position meter to avoid false measurements due to this inherent characteristic of the element.

To measure the necessary parameters for the first diagnosis, the initial conditions required for the device to function in the best possible conditions must be met. The ankle must be secured to the respective support to avoid movement thereof, an angle of 90° between femur and tibia must be guaranteed and therefore the support designed for this purpose must be adapted to the patient. Moreover, it is advisable to connect the electromyography equipment in order to verify that the muscles are at rest and thus ensure measurement only of the PCL.

Once the initial protocol has been completed the measuring device is actuated and begins to produce a rotation movement slowly on both feet; as the feet turn, so does the potentiometer, which forces said potentiometer to change its resistance, said value preferably being stored continuously. Similarly said load cells measure the force necessary to produce the rotational movement of the tibia about its own axis on each leg and thus have a numeric parameter of the opposing force produced by each of the ligaments, both in the sound knee and in the knee in question.

After obtaining these two numeric values, they are preferably converted to physical parameters as they are actually in form of two voltage measurements. However, it is not essential that said conversion be carried out and a health professional can also perform an effective analysis using the voltage measurements.

Preferably, a screen is available to observe in real time and in graphical form the comparison between the angle of rotation and force where the values obtained for a sound ligament and for the ligament in question are compared.

For a better understanding of the invention, the accompanying drawings show an embodiment of the present invention for explanatory, but not limiting, purposes.
Fig. 1 is a front perspective view of a preferred embodiment.
Fig. 2 is a detail of a means for fastening the feet in a preferred embodiment.
Fig. 3 shows the form of fastening to obtain the best results.
Fig. 4 is a side view of a preferred embodiment.
Fig. 5 is a front view of the preferred embodiment with the fastenings prepared for the procedure.
Fig. 6 shows one of the output graphs of a preferred embodiment (torque vs time).
Fig. 7 shows one of the output graphs of a preferred embodiment (angle vs time).
Fig. 8 shows a graph comparing the results for both knees.
Fig. 9 shows a graph comparing the results for both knees, one of which is injured.
Fig. 10 shows another optional embodiment.

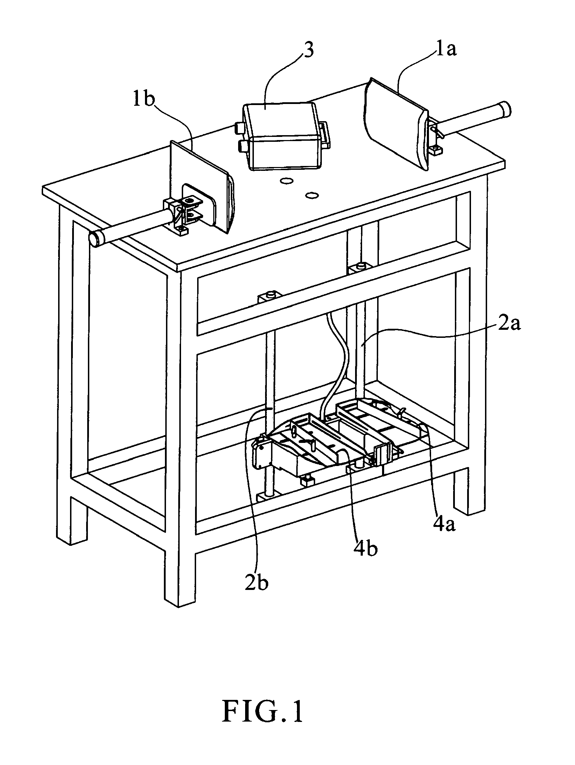

Fig. 1 shows a preferred embodiment of the present invention. In particular, among the necessary conditions, a means of positioning the femur and tibia so that they form a right angle is required and accordingly there are two bars -2a- and -2b- to adjust the height of the fastening means of the feet -4a-, -4b-, as well as means of holding the thighs -1a-, -1b- at the required angle. Another requirement consists of preventing the foot from moving sideways. There should therefore be means available to keep it straight and also ensure that it does not move, as movement would actuate one of the muscle groups of the leg preventing the process from being carried out correctly. Accordingly there is a plate which can be adjusted to the size of the patient's feet -4a-, -4b-, as well as straps to assist in immobilising the ankle. And finally, to hold the femur straight in relation to the horizontal plane and the thighs parallel to one another, a limit stop -3- is placed between the thighs.

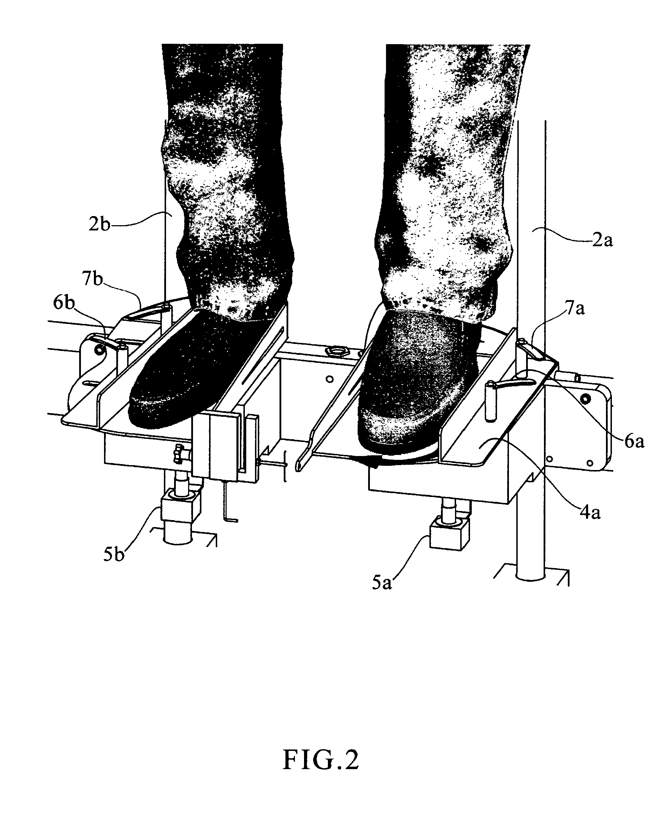

Fig. 2 shows in detail an example of the means of holding the feet correctly using an acceptable reference. The means for fastening the feet has two bars -2a-, -2bused to ensure that the angle between the tibia and femur is correct (in this particular case 90°) by moving the foot support plate -4a-, -4b-. It also has handles -6a-, -6b-, -7a-, -7b- to guarantee an initial position of the foot at 0°, and for there to be a suitable reference, as shown in the figure, said clamps have to be adjusted to position the foot at a valid point for use as a reference. And finally, the potentiometers -5a-, -5bused to measure the angular position of the tibia rotating in relation to the femur are shown.

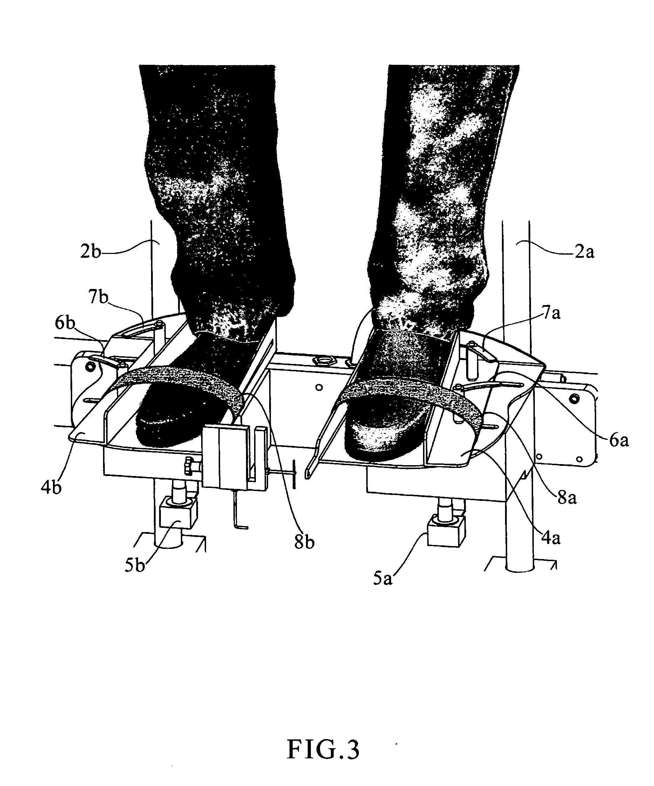

Fig. 3 shows the supports described in the previous figure in the configuration to maintain the feet in the correct position ready to begin the procedure. Additional fastening straps -8a-, -8b- are also shown which help to hold the foot on the plate -4a-, -4b-, improving the measurement obtained.

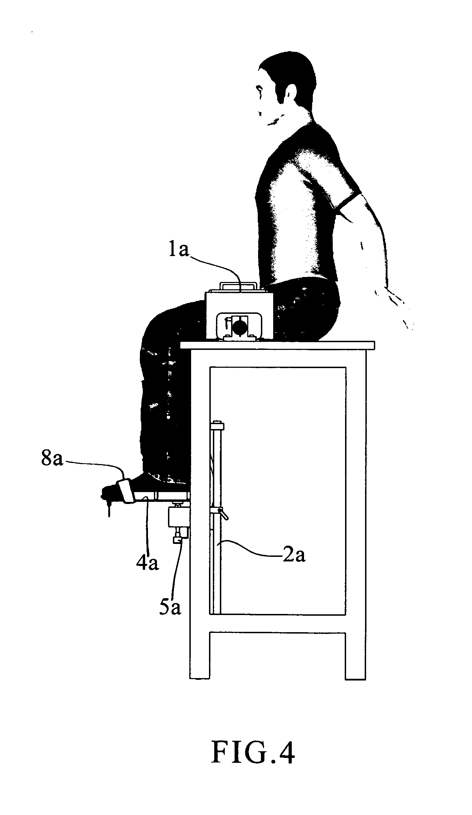

Fig. 4 shows a side view of the components required for measurement. The basic requirement is that the initial position of the feet must be at 90° to the tibia to guarantee an optimal reference and the plates -4a- and -4b- must be parallel to the thigh to avoid action in the muscle groups of the leg.

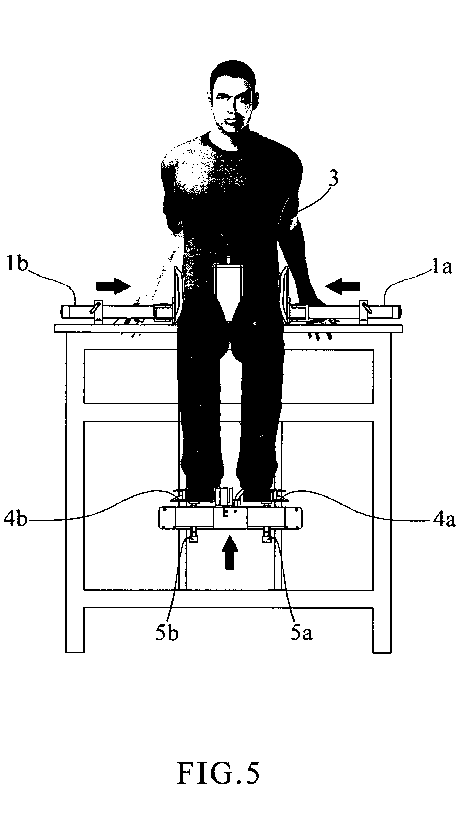

Fig. 5 shows the position of the patient and the initial adjustments preferably carried out for a successful procedure. The thigh supports -1a-, -1b-should be positioned so that they press the thigh and keep it straight and in addition a limit stop -3- should be placed to hold both femurs parallel and keep the femur straight relative to the horizontal. To support and position the feet, suitable adjustments should be made at -4a-, -4b- so that the feet are at 90° to the tibia and, preferably, they should be straight, as described in Fig. 4, to guarantee a better reference.

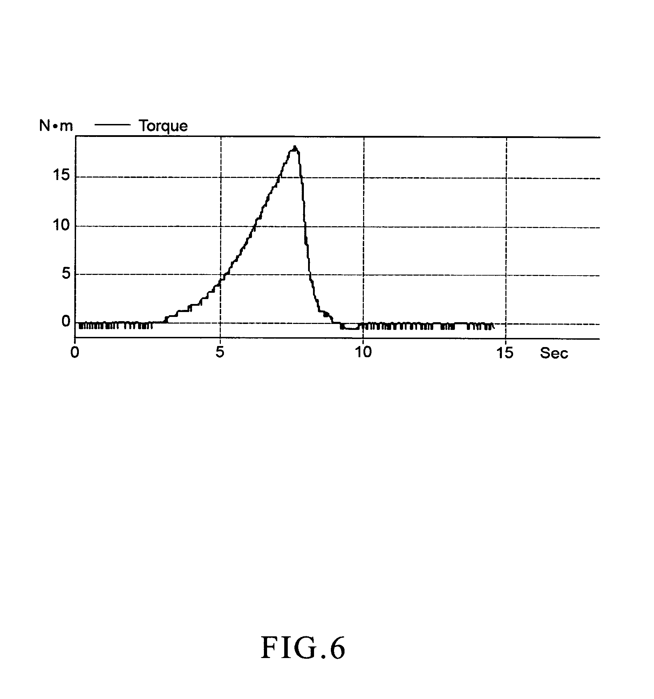

Fig. 6 shows the graph produced from the torque applied to the tibia relative to the diagnosis time. It is obtained using a signal processing system and applying a conversion factor to the value obtained by the load cell measurement in a manner widely known in the prior art. Said values are transmitted to a recording system (PC, PLC, oscilloscope, etc.), for the storage, the above-mentioned signal processing and subsequent display of the data either in real time or on completing the analysis.

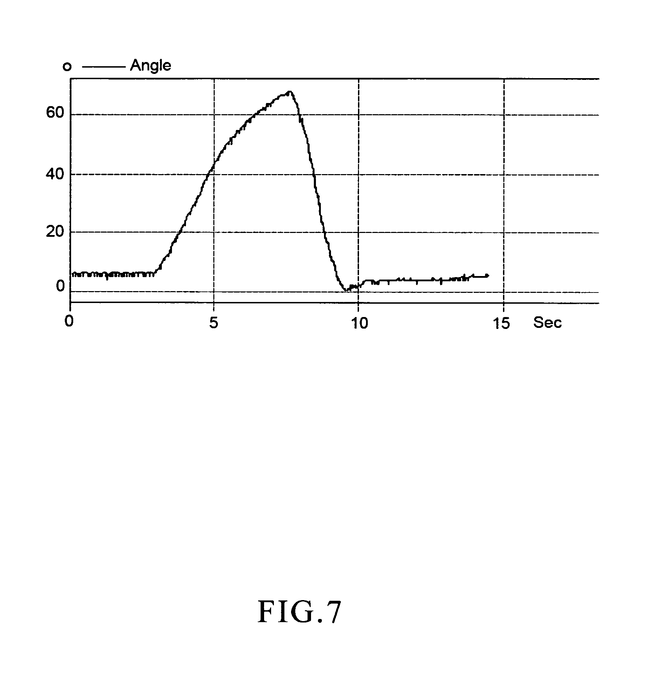

Fig. 7 shows the graph resulting from the angle of rotation of the tibia relative to the diagnosis time. It is obtained using a signal processing system and applying a conversion factor to the value obtained by said potentiometers in a manner widely known in the prior art.

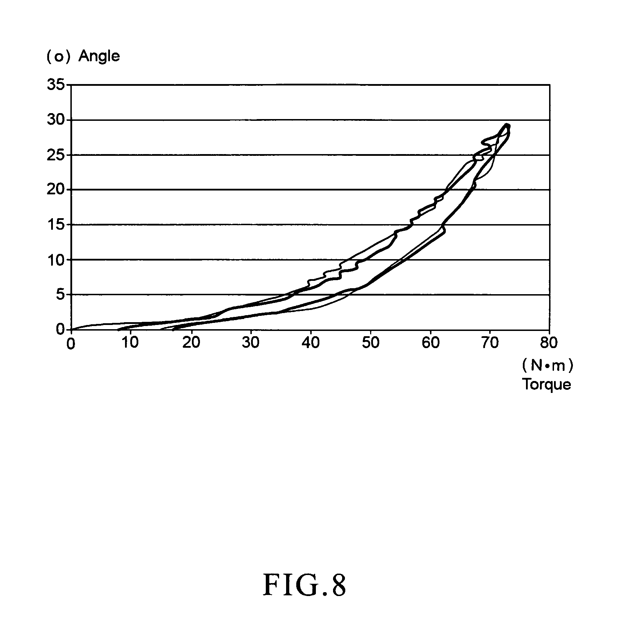

Fig. 8 shows another form of display which is also useful to determine the condition of the PCL, on this occasion in the form of graph preferably showing the behaviour of both knees simultaneously, one illustrated by the fine line and the other by the thick line. This graph shows normal operating conditions of both knees with no damage to the PCL.

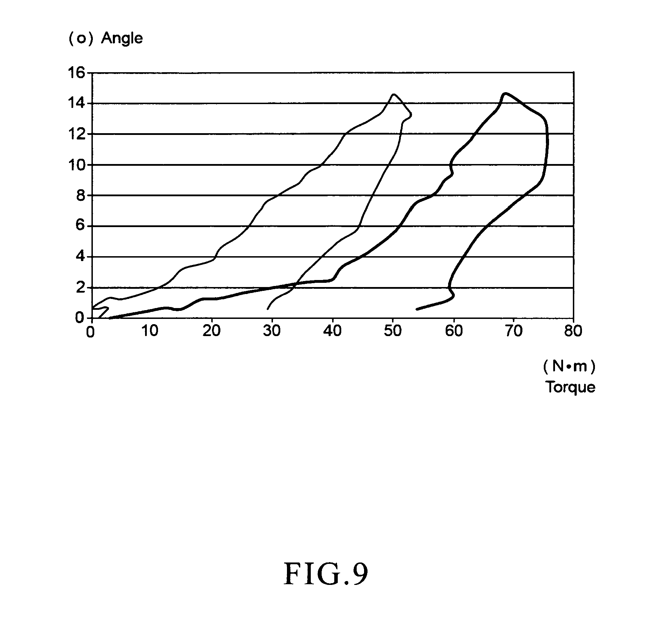

Fig. 9 is an illustration comparing the same parameters as the previous figure but with one sound knee and one knee with an injury to the PCL, where the fine line represents the affected knee and the thick line represents the sound knee. It can clearly be seen that for the knee represented by the thicker line a greater momentum of force is required (for example, at 15° about 68 Nm is required) to deform the PCL whereas for the knee shown by the fine line a far smaller momentum of force is required (about 48 Nm) which would indicate clear damage to the ligament of the knee represented by the fine line.

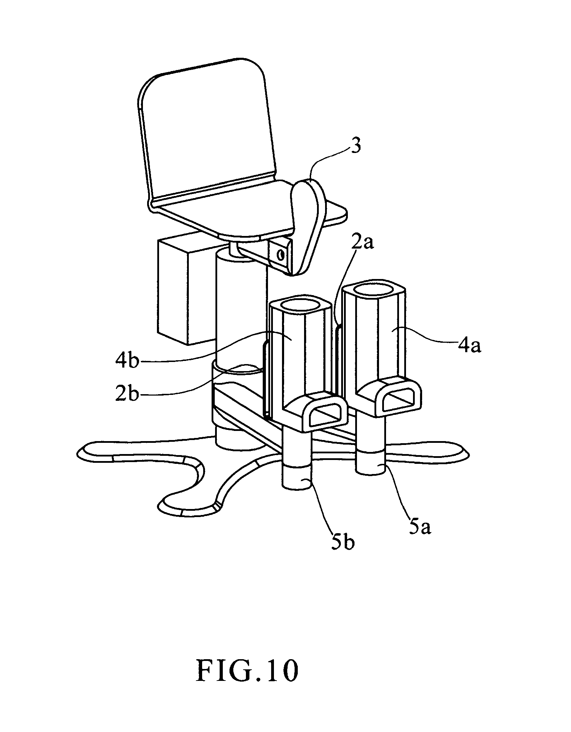

Fig. 10 shows the components of another embodiment of the same device where the principal differences which can be seen are the means to guarantee the respective angles between the foot, tibia and femur -4a-, -4b- are more restrictive and have a more stable metal structure which ensures that measuring errors are smaller. Another marked difference is that the limit stop -3- for holding the femur straight relative to the horizontal forms part of the complete unit and there are therefore no separate parts in the device.

Although the invention has been described in relation to preferred embodiments, these should not be considered as limiting the invention, which is defined by the following claims.

## Claims

1. Device for determining the condition of knee ligaments, **characterised in that** it comprises at least a means of applying force to rotate the tibia about its own axis, at least a means of measuring the angle of rotation of the tibia and at least a means of measuring the momentum of the force applied to produce said rotation.

2. Device according to claim 1, **characterised in that** the means used to apply a momentum of rotational force comprises an electric motor.

3. Device according to claim 1, **characterised in that** said means of measuring the angle comprises a potentiometer.

4. Device according to claims 1 to 3, **characterised in that** said means of measuring force comprises a load cell.

5. Device according to any one of the preceding claims, **characterised in that** it comprises a device for data acquisition, signal processing and display.

6. Device according to claim 5, **characterised in that** it comprises a screen for displaying data in real time.

7. Device according to any one of the preceding claims, **characterised in that** it comprises retention means to maintain a right angle between the tibia and the femur.

8. Device according to any one of the preceding claims, **characterised in that** it comprises retention means to maintain a right angle between the tibia and the foot.

9. Device according to claim 7, **characterised in that** said retention means comprise at least a rod on which at least a clamp is positioned, said rod having at its end a plate for positioning the patient's feet, it being possible to change the position of the pressure point of the clamp on the rod in order to adjust the rod to the length of the patient's leg.

10. Device according to any one of the preceding claims, **characterised in that** it comprises a device to avoid movements of the ankle joint.

11. Device according to any one of the preceding claims, **characterised in that** it comprises electromyography equipment to observe the state of muscular tension of the leg and/or thigh.

12. Device according to any one of the preceding claims, **characterised in that** it also comprises an emergency stop button to avoid harm or pain to the patient.

13. Device according to any one of the preceding claims, **characterised in that** the means of applying rotational force to the tibia can do so in either an outward or inward direction.

14. Device according to any one of the preceding claims, **characterised in that** said means of rotating the tibia comprises a speed regulator to control the speed.

15. Device according to any one of the preceding claims, **characterised in that** it comprises at least a press for holding the patient's thigh.

16. Device according to claim 15, **characterised in that** said press comprises at least a limit stop positioned between the thighs to avoid adduction.

17. Device according to any one of the preceding claims, **characterised in that** it comprises a plate to support and position the foot and clamps for adjusting the device to the size of the foot and avoiding movement of the joint.
